# EUROPEAN PATENT SPECIFICATION

(11) **EP 4 249 491 B1**
(45) Date of publication and mention of the grant of the patent: **04.11.2026**
(21) Application number: 23189905.5
(22) Date of filing: 09.04.2020
(51) Int. Cl.: C07D 493/08

(54) **PROCESS FOR PREPARING BICYCLIC ENOLETHER**
VERFAHREN FÜR DIE HERSTELLUNG VON BICYCLISCHENOLETHER
PROCÉDÉ POUR LA PRÉPARATION D'ÉNOL ÉTHER BICYCLIQUE

(30) Priority: 15.04.2019 EP 19169290
(43) Date of publication of application: 27.09.2023
(62) Document number(s) of the earlier application(s) in accordance with Art. 76 EPC: 20717200.8 / 3 870 587
(73) Proprietor: Firmenich SA, 1242 Satigny (CH)
(72) Inventor: JACOBY, Denis, 1242 Satigny (CH); GANDARA, Catherine, 1242 Satigny (CH)
(74) Representative: dsm-firmenich IP

(56) References cited:
- EP-A1- 3 199 514
- EP-A1- 3 862 356
- WO-A1-2018/011386
- WO-A1-2019/086207
- WO-A1-2020/150454

## Description

### Technical field

The present invention relates to the field of organic synthesis and more specifically it concerns a process for preparing compound of formula (I) catalyzed by a metal complex.

### Background

The bicyclic enol ether derivative of formula (I) is typical intermediate toward more valuable compounds, such as saturated or unsaturated cyclic ketones. For examples, 16-oxabicyclo[10.3.1]hexadec-12-ene or 14-methyl-16-oxabicyclo[10.3.1]hexadec-12-ene are key intermediates toward highly appreciated perfumery ingredients such as Exaltenone or Muscenone^{®} (trademark from Firmenich SA). Said intermediates have been obtained since decades thanks to the double reduction of a macrocyclic dione; e.g. 1,5-cyclopentadecanedione or 3-methylcyclopentadecane-1,5-dione, into the corresponding macrocyclic diol; e.g. 1,5-cyclopentadecanediol or 3-methylcyclopentadecane-1,5-diol, followed by dehydrogenation and dehydration to form a macrocyclic enol ether. The direct enol ether formation from the dione starting material has never been reported.

So, there is still a need to develop such a more straightforward approach toward bicyclic enolether while improving the yield. Reference is made to WO2019/086207, WO2028/011386, EP3199514, EP3862356 and WO2020/150454.

The present invention allows obtaining compound of formula (I) starting from cyclic diketone of formula (II) under transfer hydrogenation conditions using a secondary alcohol as a source of hydrogen.

### Summary of the Invention

The invention relates to a novel process allowing the preparation of compound of formula (I) starting from compound of formula (II) while avoiding the formation and isolation step of the corresponding diol.

So, the object of the present invention is a process for the preparation of a compound of formula (I) 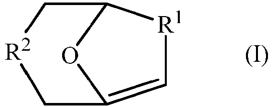
in a form of any one of its stereoisomers or a mixture thereof and wherein R¹ represents a linear or branched C₁₋₅ alkanediyl or alkenediyl group, optionally substituted by a phenyl group and R² represents a linear or branched C₁₋₁₀ alkanediyl or alkenediyl group, optionally substituted by a phenyl group;
comprising the reaction of a compound of the formula (II) 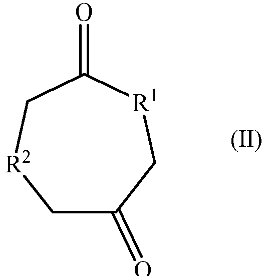
in a form of any one of its stereoisomers and wherein R¹ and R² have the same meaning as defined in formula (I); wherein the process is a one-pot reduction and cyclisation-dehydration type reaction of compound of formula (II) under transfer hydrogenation conditions;
with metal catalyst, provided that ruthenium catalyst is excluded, and in the presence of a hydrogen source;
wherein the metal catalyst is present in a concentration value ranging from 1000 to 15000 ppm relative to the total amount of substrate.

### Description of the invention

Surprisingly, it has now been discovered that the compound of formula (I) can be produced in an advantageous manner by means of a one-pot reduction and cyclisation-dehydration type reaction of compound of formula (II) under transfer hydrogenation conditions. The invention's conditions allow avoiding the difficult handling of the corresponding diol and are highly selective toward monoreduction. The invention's process leads to the formation of a bicyclic enol ether in high yield without compromise on the conversion. In addition, the invention's process uses a catalytic system which may be recycled and circumventing the use of toxic reagent such as borohydride, rendering said transformation more sustainable.

Therefore, a first object of the present invention is process for the preparation of a compound of formula (I) 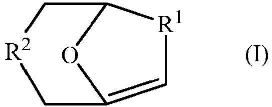
in a form of any one of its stereoisomers or a mixture thereof and wherein R¹ represents a linear or branched C₁₋₅ alkanediyl or alkenediyl group, optionally substituted by a phenyl group and R² represents a linear or branched C₁₋₁₀ alkanediyl or alkenediyl group, optionally substituted by a phenyl group;
comprising, or consisting of, the reaction of a compound of the formula (II) 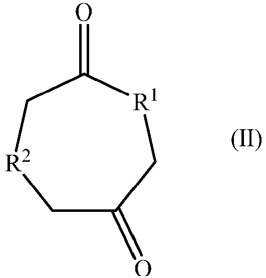
in a form of any one of its stereoisomers and wherein R¹ and R² have the same meaning
as defined in formula (I); wherein the process is a one-pot reduction and cyclisation-dehydration tape reaction of compound of formula (II) under transfer hydrogenation conditions;
with a metal catalyst, provided that ruthenium catalyst is excluded, and in the presence of a hydrogen source; wherein the metal catalyst is present in a concentration value ranging from 1000 ppm to 15000 ppm relative to the total amount of substrate.

According to any embodiments of the invention, the invention process is a one step process.

According to any embodiments of the invention, the invention process is a full conversion process.

By "full conversion process", it is meant the normal meaning in the art; i.e. the invention's process is performed until complete transformation of compound of formula (II).

According to any embodiments of the invention, a compound of formula (III) 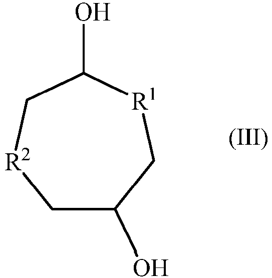 in a form of any one of its stereoisomers and wherein R¹ and R² have the same meaning as defined in formula (I);
is not formed.

In other words, the invention's process allows obtaining compound of formula (I) in one step without the formation of the double reduction product of formula (III). The invention's process allows obtaining mainly compound of formula (I) without the formation of the undesired over-hydrogenated diol of formula (III); i.e. the invention's process is highly selective.

According to any embodiments of the invention, and independently of the specific aspects, the compound (I) as well as the corresponding compound (II) can be in the form of any one of its stereoisomers or mixture thereof. For the sake of clarity by the term stereoisomer it is intended any diastereoisomers, enantiomers, racemate.

Indeed, the compound (I) or (II) may have at least one stereogenic center which can have different stereochemistry (i.e. when two stereogenic centers are present, compound (I) or (II) can have (R,R) or (R,S) configuration). Each of said stereogenic centers can be in a relative or absolute configuration R or S or a mixture thereof or in other words said compound of formula (II) or (I) can be in a form of pure enantiomer or diastereoisomer, or in a form of a mixture of stereoisomers.

According to any one of the above embodiments of the invention, said compounds of formula (II) are C₇-C₂₀ compounds.

According to any one of the above embodiments of the invention, compound (I) may be a compound of formula 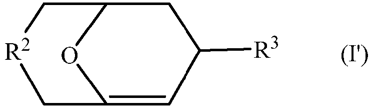 in a form of any one of its stereoisomers and wherein R² has the same meaning as defined in formula (I) and R³ represents a hydrogen atom or a methyl group.

According to any one of the above embodiments of the invention, compound (II) may be a compound of formula 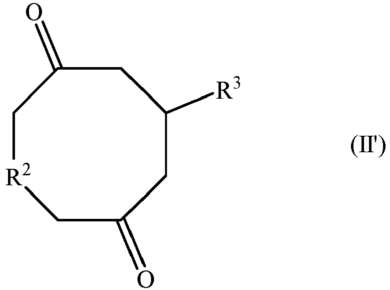 in a form of any one of its stereoisomers and wherein R² has the same meaning as defined in formula (I) and R³ represents a hydrogen atom or a methyl group.

According to any embodiments of the invention, R¹ represents linear or branched C₁₋₅ alkanediyl or alkenediyl group optionally substituted by a phenyl group. Preferably, R¹ represents a linear or branched C₁₋₄ alkanediyl group. Preferably, R¹ represents a linear or branched C₂₋₃ alkanediyl group. Even more preferably, R¹ represents a 1,2-propanediyl group or a 1,2-ethanediyl group. Even more preferably, R¹ represents a 1,2- propanediyl group.

According to any embodiments of the invention, R² represents a linear or branched C₁₋₁₀ alkanediyl or alkenediyl group optionally substituted by a phenyl group. Preferably, R² represents a linear or branched C₁₋₁₀ alkanediyl group. Preferably, R² represents a linear or branched C₄₋₉ alkanediyl group. Preferably, R² represents a linear or branched C₆₋₉ alkanediyl group. Even more preferably, R² represents a 1,8-octanediyl group.

According to any embodiments of the invention, R³ represents a hydrogen atom or a methyl group. Preferably, R³ may represent a methyl group.

Non-limiting examples of suitable compounds of formula (I) may include 12-oxabicyclo[6.3.1]dodec-8-ene, 10-methyl-12-oxabicyclo[6.3.1]dodec-8-ene, 13-oxabicyclo[7.3.1]tridec-9-ene, 11-methyl-13-oxabicyclo[7.3.1]tridec-9-ene, 14-oxabicyclo[8.3.1]tetradec-10-ene, 12-methyl-14-oxabicyclo[8.3.1]tetradec-10-ene, 15-oxabicyclo[9.3.1]pentadec-11-ene, 13-methyl-15-oxabicyclo[9.3.1]pentadec-11-ene, 16-oxabicyclo[10.3.1]hexadec-12-ene, 14-methyl-16-oxabicyclo[10.3.1]hexadec-12-ene, 17-oxabicyclo[11.3.1]heptadec-13-ene or 15-methyl-17-oxabicyclo[11.3.1]heptadec-13-ene. Preferably, the compound of formula (I) may be 16-oxabicyclo[10.3.1]hexadec-12-ene, 14-methyl-16-oxabicyclo[10.3.1]hexadec-12-ene, 17-oxabicyclo[11.3.1]heptadec-13-ene or 15-methyl-17-oxabicyclo[11.3.1]heptadec-13-ene. Preferably, the compound of formula (I) may be 16-oxabicyclo[10.3.1]hexadec-12-ene or 14-methyl-16-oxabicyclo[10.3.1]hexadec-12-ene. Even more preferably, the compound of formula (I) may be 14-methyl-16-oxabicyclo[10.3.1]hexadec-12-ene.

Non-limiting examples of suitable compounds of formula (II) may include cycloundecane-1,5-dione, 3-methylcycloundecane-1,5-dione, cyclododecane-1,5-dione, 3-methylcyclododecane-1,5-dione, cyclotridecane-1,5-dione, 3-methylcyclotridecane-1,5-dione, cyclotetradecane-1,5-dione, 3-methylcyclotetradecane-1,5-dione, cyclopentadecane-1,5-dione, 3-methylcyclopentadecane-1,5-dione, cyclohexadecane-1,5-dione or 3-methylcyclohexadecane-1,5-dione . Preferably, the compound of formula (II) may be cyclopentadecane-1,5-dione, 3-methylcyclopentadecane-1,5-dione, cyclohexadecane-1,5-dione or 3-methylcyclohexadecane-1,5-dione. Preferably, the compound of formula (II) may be cyclopentadecane-1,5-dione or 3-methylcyclopentadecane-1,5-dione. Even more preferably, the compound of formula (II) may be 3-methylcyclopentadecane-1,5-dione.

The compound of formula (II) is commercially available compound or can be prepared by several methods, such as the one reported in Helvetica Chimica Acta 1967, 50, 705, or in WO2016104474 or in WO2016184948.

According to any embodiments of the invention, the catalyst is a metal catalyst. Preferably, the metal catalyst may be of formula

[M(O)ₘ(X)ₙ] (IV)

wherein M is a metal selected from the group consisting of transition metal or post-transition metal, m is 0, 1 or 2, X represents an anionic ligand; and n is an integer between 1 to 6.

According to any of the above embodiments of the invention, M may be a metal selected from the group consisting of metals of column IIIA (such as Al), IVA, IIIB, IVB (such as Ti or Zr), VB (such as V), VIB and VIIB of the periodic table. Preferably, the M may be selected from the group consisting of Aluminum, Titanium, Zirconium and Vanadium.

According to any of the above embodiments of the invention, n may be an integer between 1 and 4. Preferably, n may be an integer between 2 and 4. Even more preferably, n may be 3 or 4.

According to any of the above embodiments of the invention, X represents an anionic ligand. A non-limiting list of anionic ligand includes a hydrogen or halogen atom, a hydroxy, an oxy or betadiketonato group, or an alkoxy or carboxylic radical.

By the term "betadiketonato", it is meant the normal meaning in the art; i.e a group of formula R^{a}(COCHCO)⁻R ^{a} wherein R^{a} are, independently from each other, a C₁ to C₁₈ alkyl group and the negative charge is delocalized.

According to any of the above embodiments of the invention, in formula (III), each X represents, simultaneously or independently, a hydrogen or fluorine, chlorine, bromine or iodine atom, a hydroxy or acetylacetonate group, a C₁ to C₁₈ alkoxy radical, such as a methoxy, ethoxy or isopropoxy, *sec*-butoxy radical, or a C₁ to C₁₈ carboxylic radical such as a HCOO, CH₃COO, CH₃CH₂COO or phenylCOO radical. Preferably, each X represents, simultaneously or independently, a hydroxy, methoxy, ethoxy, propoxy, butoxy, isopropoxy, or acetylacetonate radical.

Non-limiting examples of suitable metalcatalyst of formula (III) may include[Al(OR)₃], [Al(OAc)₃], [Al(acac)₃], [Ti(OR)₄], [Ti(O)(acac)₂], [Zr(OR)₄], [Zr(acac)₄], [V(O)(acac)₂], [V(acac)₃], [V(O)(OR)₃]; R being a C₁₋₆ alkyl group, preferably R being a C₁₋₄ alkyl group.

The metal catalyst can be added into the reaction medium of the invention's process in a large range of concentrations. As non-limiting examples, one can cite as metal concentration values those ranging from 1000 ppm to 100000 ppm, relative to the total amount of substrate. Preferably, the metal concentration will be comprised between 5000 ppm to 15000 ppm. It goes without saying that the process works also with more catalyst. However the optimum concentration of metal will depend, as the person skilled in the art knows, on the nature of the latter, on the nature of the substrate, on the temperature and on the desired time of reaction.

According to any one of the above embodiments of the invention, the hydrogen source is a hydrocarbon comprising at least one secondary alcohol functional group and having a boiling point equal to or grater than 80°C, preferably equal to or greater than 110°C, even more preferably, equal to or greater than 120°C. Said hydrogen source borrows hydrogen while generating ketone. The borrowing hydrogen source may be a secondary alcohol. In particular, the borrowing hydrogen source may be of formula 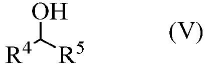 wherein R⁴ and R⁵, independently from each other, represent a C₁₋₁₀ linear alkyl group optionally substituted by a hydroxy group or an aryl group, a C₂₋₁₀ linear alkenyl group optionally substituted by a hydroxy group or an aryl group, a C₃₋₁₀ branched or cyclic alkyl or alkenyl group optionally substituted by a hydroxy group or an aryl group, or a phenyl group optionally substituted by one to five C₁₋₃ alkyl or alkoxy groups, hydroxy groups or halogen atoms; or R⁴ and R⁵, when taken together, represent a C₂₋₁₀ linear or branched alkanediyl or alkenediyl optionally substituted by a hydroxy group or an aryl group. The hydrogen source of formula (V) is a C₄₋₁₀ compound.

The term "aryl group" designates the normal meaning in the art; i.e. an aromatic hydrocarbon group such as phenyl or naphthyl group optionally substituted. Non-limiting examples of the optional substituent of the aryl group may include C₁₋₃ alkyl or alkoxy group, a hydroxy group or a halogen atom.

According to any one of the above embodiments, R⁴ may represent a C₁₋₁₀ linear alkyl group optionally substituted by a hydroxy group or an aryl group or a C₃₋₁₀ branched or cyclic alkyl group optionally substituted by a hydroxy group or an aryl group, or a phenyl group optionally substituted by one to five C₁₋₃ alkyl or alkoxy group, a hydroxy group or a halogen atom. Preferably, R⁴ may represent a C₁₋₁₀ linear alkyl group optionally substituted by a hydroxy group or a C₃₋₁₀ branched or cyclic alkyl group optionally substituted by a hydroxy group. Preferably, R⁴ may represent a C₃₋₈ linear or branched alkyl group optionally substituted by a hydroxy group. Even more preferably, R⁴ may represent a methyl, ethyl, propyl, isopropyl, butyl, octyl group optionally substituted by a hydroxy group.

According to any one of the above embodiments, R⁵ may represent a C₁₋₁₀ linear alkyl group optionally substituted by a hydroxy group or an aryl group or a C₃₋₁₀ branched or cyclic alkyl group optionally substituted by a hydroxy group or an aryl group. Preferably, R⁵ may represent a methyl, an ethyl or a propyl group.

According to any one of the above embodiments, R⁴ and R⁵, when taken together, may represent a C₄₋₇ linear, branched alkanediyl or alkenediyl optionally substituted by a hydroxy group. Preferably, R⁴ and R⁵, when taken together, may represent a C₄₋₇ linear alkanediyl. Even more preferably, R⁴ and R⁵, when taken together, may represent a C₄₋₅ linear alkanediyl.

Non-limiting example of suitable hydrogen source may include 1-phenylethan-1-ol, 2-methyl-2,4-pentanediol, cyclohexanol, 4-methylpentan-2-ol, cyclopentanol or octan-2-ol. Preferably, the hydrogen source may be cyclopentanol, or 4-methylpentan-2-ol.

The hydrogen source can be added into the reaction medium of the invention's process in a large range of concentrations. As non-limiting examples, one can cite as hydrogen source concentration values those ranging from 1 equivalent to 50 equivalents, or even between 1 equivalent to 5 equivalents, relative to the amount of compound of formula (II). It goes without saying that the optimum concentration of hydrogen source will depend, as the person skilled in the art knows, on the nature of the latter, on the nature of the substrate, of the temperature and on the catalyst used during the process, as well as the desired time of reaction.

According to any embodiments of the invention, the invention process may be performed in the presence of a co-catalyst. Non-limiting examples of suitable co-catalyst include tertiary amine, such as pyridine, bis-pyridine, trimethylamine, lutidine, *N*,*N-*Diisopropylethylamine or 1,8-Diazabicyclo[5.4.0]undec-7-ene or tertiary phosphine, or phenols optionally substituted, or bis phenols optionally substituted. Preferably, the cocatalyst may be a tertiary amine or a pyridine or a substituted bis phenol. Even more preferably, the cocatalyst may be a methylpyridine or a dihydroxy biphenyl ingredient such as 2,2'-dihydroxydiphenylmethane, 2,2'-dihydroxybiphenyl.

The co-catalyst can be added into the reaction medium of the invention's process in a large range of concentrations. As non-limiting examples, one can cite as co-catalyst concentration values those ranging from 0.1 equivalent to 2 equivalent relative to the catalyst, It goes without saying that the optimum concentration of the co-catalyst will depend, as the person skilled in the art knows, on the nature of the latter, on the nature of the substrate, of the temperature and on the catalyst used during the process, as well as the desired time of reaction.

The invention's process is carried out under batch or continuous conditions.

The reaction can be carried out in the absence of a solvent.

The temperature of the invention's process may be comprised between 120°C and 300°C, more preferably in the range comprised between 150 °C and 250°C. Of course, a person skilled in the art is also able to select the preferred temperature as a function of the melting and boiling point of the hydrogen source and of the starting and final products as well as the desired time of reaction or conversion.

The invention's process may be performed under atmospheric pressure or under a slight vacuum. The invention's process may be performed under inert atmosphere such as nitrogen or argon.

According to any one of the above embodiments of the invention, the more volatile compounds generated during the invention's process are removed continuously during the invention's process. The removal of said more volatile compounds such as water and ketone formed during the invention process may be distillated during the invention process.

### Examples

The invention will now be described in further details by way of the following examples, wherein the abbreviations have the usual meaning in the art, the temperatures are indicated in degrees centigrade (°C); the NMR spectral data were recorded in CDCl₃ (if not stated otherwise) with a 360 or 400 MHz machine for ¹H and ¹³C, the chemical shifts δ are indicated in ppm with respect to TMS as standard, the coupling constants J are expressed in Hz.

### Example 1

### Preparation of compound of formula (I) starting from compound of formula (II)

In 1l glass reactor equipped with a mechanical stirrer, a packed column, a reflux condenser, 250g of 3-methyl-1,5-cyclopentadecanedione (0.99mol), 50g of the hydrogen source (see Table 1) and V(acac)₃ (17.2g, 0.049mol), were loaded at atmospheric pressure. The mixture was stirred and heated to reflux then the rest of the hydrogen source (450g) was dosed over 22h while distilling into a flask the light fraction that was formed during the reaction (water and resulting ketone). The resulting mixture was cooled to 100°C and the remaining solvent was concentrated under vacuum. The residual oil (230g) was flash distilled (170°C, 1mbar) and the distillate was analyzed by GC (see Table 1).

**Table 1: Preparation of compound of formula (I) from compound of formula (II)**

| Hydrogen source | Amount of hydrogen source (g) | Reaction temperature (°C) | Reaction pressure (mbar) | Compound (I) (%) | Compound (II) (%) | Yield (%)²⁾ |
|---|---|---|---|---|---|---|
| 4-Methylpentan 2-ol¹⁾ | 500 | 170 | 1000 | 89.1 | 3 | 85.6 |
| Cyclopentanol¹⁾ | 500 | 175 | 1000 | 89.6 | 1 | 89.7 |

| | | | | | | |
|---|---|---|---|---|---|---|
| 1) pre loading of 10% of the total charge then addition of the rest over 16h. 2) mainly full conversion and high selectivity. | | | | | | |

### Example 2

### Preparation of compound of formula (I) starting from compound of formula (II)

In 1l glass reactor equipped with a mechanical stirrer, a packed column, a reflux condenser, 250g of 3-methyl-1,5-cyclopentadecanedione (0.99mol), 50g of cyclohexanol, Zr(OPr)₄ (4.63g, 0.0098mol, 70% in PrOH), and 2,2'-dihydroxydiphenylmethane (1.59g, 0.0098mol) were loaded at atmospheric pressure. The mixture was stirred and heated to reflux then the rest of the hydrogen source (450g) was dosed over 22h while distilling into a flask the light fraction that was formed during the reaction (water, cyclohexanol, cyclohexanone). The resulting mixture was cooled to 100°C and the remaining solvent was concentrated under vacuum. The residual oil (242g) was flash distilled (170°C, 1mbar) and the distillate was analyzed by GC (compound I 92.7% purity, 91.2% yield). 3-methyl-1,5-cyclopentadecanediol was not formed and 3-methyl-1,5-cyclopentadecanedione was completely converted.

### Example 3

### Preparation of compound of formula (I) starting from compound of formula (II)

In 1l glass reactor equipped with a mechanical stirrer, a packed column, a reflux condenser, 250g of 3-methyl-1,5-cyclopentadecanedione (0.99mol), 50g of 2-Octanol, Zr(OPr)₄ (9.26g, 0.0196mol, 70% in PrOH), and 2,2'-dihydroxybiphenyl (3.7g, 0.0196mol) were loaded at 200 mbar. The mixture was stirred and heated to reflux then the rest of the hydrogen source (250g) was dosed over 16h while distilling into a flask the light fraction that was formed during the reaction (water, 2-Octanol, 2-Octanone). The resulting mixture was cooled to 100°C and the remaining solvent was concentrated under vacuum. The residual oil (230g) was flash distilled (180°C, 1mbar) and the distillate was analyzed by GC (compound I 89.2% purity, 94.1% yield). 3-methyl-1,5-cyclopentadecanediol was not formed and 3-methyl-1,5-cyclopentadecanedione was completely converted.

### Example 4

### Preparation of compound of formula (I) starting from compound of formula (II)

In 1l glass reactor equipped with a mechanical stirrer, a packed column, a reflux condenser, 250g of 3-methyl-1,5-cyclopentadecanedione (0.99mol), 50g of the hydrogen source (see Table 2), Ti(OiPr)₄ (5.6g, 0.02mol), and 2,2'-dihydroxybiphenyl (3.7g, 0.02mol) were loaded at a pressure indicated in the table 2. The mixture was stirred and heated to reflux then the rest of the hydrogen source (450g) was dosed over 22h while distilling into a flask the light fraction that was formed during the reaction (water and resulting ketone). The resulting mixture was cooled to 100°C and the remaining solvent was concentrated under vacuum. The residual oil (≈230g) was flash distilled (170°C, 1mbar) and the distillate was analyzed by GC (see Table 2).

**Table 2: Preparation of compound of formula (I) from compound of formula (II)**

| Hydrogen source | Amount of hydrogen source (g) | Reaction temperature (°C) | Reaction pressure (mbar) | Compound (I) (%) | Compound (II) (%) | Yield (%) |
|---|---|---|---|---|---|---|
| 4-Methylpentan 2-ol¹⁾ | 500 | 170 | 1000 | 88.5 | 1.35 | 95.6 |
| Cyclopentanol¹⁾ | 500 | 175 | 1000 | 84.1 | 0.9 | 95.2 |
| Cyclohexanol¹⁾ | 500 | 170 | 450 | 83.8 | 1.35 | 93 |

| | | | | | | |
|---|---|---|---|---|---|---|
| 1) pre loading of 10% of the total charge then addition of the rest over 16h. 2) mainly full conversion and high selectivity. | | | | | | |

### Example 5

### Preparation of compound of formula (I) starting from compound of formula (II)

In 1l glass reactor equipped with a mechanical stirrer, a packed column, a reflux condenser, 250g of 3-methyl-1,5-cyclopentadecanedione (0.99mol), 50g of cyclopentanol, Al(OBu)₃ (12.3g, 0.05mol), and 2,2'-dihydroxydiphenylmethane (8g, 0.05mol) were loaded at atmospheric pressure. The mixture was stirred and heated to reflux then the rest of the hydrogen source (450g) was dosed over 22h while distilling into a flask the light fraction that was formed during the reaction (water, cyclopentanol, cyclopentanone). The resulting mixture was cooled to 100°C and the remaining solvent was concentrated under vacuum. The residual oil (247g) was flash distilled (170°C, 1mbar) and the distillate was analyzed by GC (compound I 85% purity, 85% yield). 3-methyl-1,5-cyclopentadecanediol was not formed and 3-methyl-1,5-cyclopentadecanedione was completely converted.

## Claims

1. A process for the preparation of a compound of formula (I) 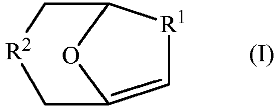
in a form of any one of its stereoisomers or a mixture thereof and wherein R¹ represents a linear or branched C₁₋₅ alkanediyl or alkenediyl group, optionally substituted by a phenyl group and R² represents a linear or branched C₁₋₁₀ alkanediyl or alkenediyl group, optionally substituted by a phenyl group;
comprising the reaction of a compound of the formula (II) 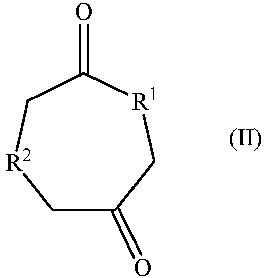
in a form of any one of its stereoisomers and wherein R¹ and R² have the same meaning as defined in formula (I);
wherein the process is a one-pot reduction and cyclisation-dehydration type reaction of compound of formula (II) under transfer hydrogenation conditions;
with a metal catalyst, provided that ruthenium catalyst is excluded, and in the presence of a hydrogen source;
wherein the metal catalyst is present in a concentration value ranging from 1000 ppm to 15000 ppm relative to the total amount of substrate.

2. The process according to claim 1, **characterized in that** R¹ represents a linear or branched C₁₋₄ alkanediyl group.

3. The process according to claim 1 or 2, **characterized in that** R¹ represents a linear or branched C₂₋₃ alkanediyl group.

4. The process according to according to any one of claims 1 to 3, **characterized in that** R¹ represents a 1,2-propanediyl group or 1,2-ethanediyl group.

5. The process according to any one of claims 1 to 4, **characterized in that** R² represents a linear or branched C₄₋₉ alkanediyl group.

6. The process according to any one of claims 1 to 5, **characterized in that** R² represents a linear or branched C₆₋₉ alkanediyl group.

7. The process according to any one of claims 1 to 6, **characterized in that** R² represents a 1,8-octanediyl group.

8. The process according to any one of claims 1 to 7, **characterized in that** compound of formula (I) may be selected from the group consisting of 12-oxabicyclo[6.3.1]dodec-8-ene, 10-methyl-12-oxabicyclo[6.3.1]dodec-8-ene, 13-oxabicyclo[7.3.1]tridec-9-ene, 11-methyl-13-oxabicyclo[7.3.1]tridec-9-ene, 14-oxabicyclo[8.3.1]tetradec-10-ene, 12-methyl-14-oxabicyclo[8.3.1]tetradec-10-ene, 15-oxabicyclo[9.3.1]pentadec-11-ene, 13-methyl-15-oxabicyclo[9.3.1]pentadec-11-ene, 16-oxabicyclo[10.3.1]hexadec-12-ene, 14-methyl-16-oxabicyclo[10.3.1]hexadec-12-ene, 17-oxabicyclo[11.3.1]heptadec-13-ene and 15-methyl-17-oxabicyclo[11.3.1]heptadec-13-ene.

9. The process according to any one of claims 1 to 8, **characterized in that** compound of formula (II) may be selected from the group consisting of cycloundecane-1,5-dione, 3-methylcycloundecane-1,5-dione, cyclododecane-1,5-dione, 3-methylcyclododecane-1,5-dione, cyclotridecane-1,5-dione, 3-methylcyclotridecane-1,5-dione, cyclotetradecane-1,5-dione, 3-methylcyclotetradecane-1,5-dione, cyclopentadecane-1,5-dione, 3-methylcyclopentadecane-1,5-dione, cyclohexadecane-1,5-dione and 3-methylcyclohexadecane-1,5-dione.

10. The process according to any one of claims 1 to 9, **characterized in that** the metal catalyst is of formula
[M(O)m(X)n] (IV)
wherein M is a metal selected from the group consisting of transition metal or post-transition metal, m is 0 or 1 or 2, X represents an anionic ligand; and when n is an integer between 1 to 6.

11. The process according to claim 10, **characterized in that** M is selected from the group consisting of Aluminum, Titanium, Zirconium and Vanadium.

12. The process according to any one of claims 1 to 11, **characterized in that** the metal catalyst is selected from the group consisting of [Al(OR)₃], [Al(OAc)₃], [Al(acac)₃], [Ti(OR)₄], [Ti(O)(acac)₂], [Zr(OR)₄], [Zr(acac)₄], [V(O)(acac)₂], [V(acac)₃] and [V(O)(OR)₃]; R being a C₁₋₆ alkyl group.

13. The process according to any one of claims 1 to 12, **characterized in that** the hydrogen source is a hydrocarbon comprising at least one secondary alcohol functional group and having a boiling point equal or greater than 80°C.

14. The process according to any one of claims 1 to 13, **characterized in that** the hydrogen source is of formula 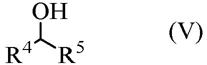 wherein R⁴ and R⁵, independently from each other, represent a C₁₋₁₀ linear alkyl group optionally substituted by a hydroxy group or an aryl group, a C₂₋₁₀ linear alkenyl group optionally substituted by a hydroxy group or an aryl group, a C₃₋₁₀ branched or cyclic alkyl or alkenyl group optionally substituted by a hydroxy group or an aryl group, or a phenyl group optionally substituted by one to five C₁₋₃ alkyl or alkoxy groups, hydroxy groups or halogen atoms; or R⁴ and R⁵, when taken together, represent a C₂₋₁₀ linear or branched alkanediyl or alkenediyl optionally substituted by a hydroxy group or an aryl group.

15. The process according to any one of claims 1 to 14, **characterized in that** the hydrogen source is 1-phenylethan-1-ol, 2-methyl-2,4-pentanediol, cyclohexanol, 4-methylpentan-2-ol, cyclopentanol or octan-2-ol.

16. The process according to any one of claims 1 to 15, **characterized in that** the process is performed in the presence of a co-catalyst, preferably the co-catalyst is phenols optionally substituted or bis phenols optionally substituted.

17. The process according to any one of claims 1 to 16, **characterized in that** compound (I) is 14-methyl-16-oxabicyclo[10.3.1]hexadec-12-ene and compound (II) is 3-methylcyclopentadecane-1,5-dione.

18. The process according to claim 17, **characterized in that** the process further comprises the step of converting 14-methyl-16-oxabicyclo[10.3.1]hexadec-12-ene into 3-methyl-5-cyclopentadecen-1-one.

## Patentansprüche

1. Prozess zur Herstellung einer Verbindung der Formel (I) 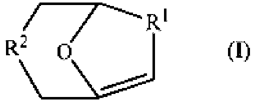
in Form eines ihrer Stereoisomere oder eines Gemischs davon, wobei R¹ eine lineare oder verzweigte C₁₋₅-Alkandiyl- oder Alkendiylgruppe darstellt, die gegebenenfalls durch eine Phenylgruppe substituiert ist, und R² eine lineare oder verzweigte C₁₋₁₀-Alkandiyl- oder Alkendiylgruppe darstellt, die gegebenenfalls durch eine Phenylgruppe substituiert ist;
umfassend die Umsetzung einer Verbindung der Formel (II) 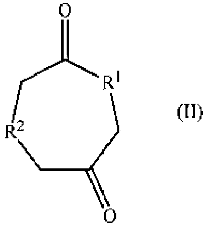
in Form eines ihrer Stereoisomere und wobei R¹ und R² die gleiche Bedeutung aufweisen, wie in Formel (I) definiert;
wobei der Prozess eine Eintopf-Reduktions- und Cyclisierungs-Dehydratisierungsreaktion der Verbindung der Formel (II) unter Transferhydrierungsbedingungen ist;
mit einem Metallkatalysator, mit der Maßgabe, dass Rutheniumkatalysator ausgeschlossen ist, und in Gegenwart einer Wasserstoffquelle;
wobei der Metallkatalysator in einer Konzentration vorhanden ist, die von 1000 ppm bis 15000 ppm im Verhältnis zur Gesamtmenge des Substrats reicht.

2. Prozess nach Anspruch 1, **dadurch gekennzeichnet, dass** R¹ eine lineare oder verzweigte C₁₋₄-Alkandiylgruppe darstellt.

3. Prozess nach Anspruch 1 oder 2, **dadurch gekennzeichnet, dass** R¹ eine lineare oder verzweigte C₂₋₃-Alkandiylgruppe darstellt.

4. Prozess nach einem der Ansprüche 1 bis 3, **dadurch gekennzeichnet, dass** R¹ eine 1,2-Propandiylgruppe oder 1,2-Ethandiylgruppe darstellt.

5. Prozess nach einem der Ansprüche 1 bis 4, **dadurch gekennzeichnet, dass** R² eine lineare oder verzweigte C₄₋₉ Alkandiylgruppe darstellt.

6. Prozess nach einem der Ansprüche 1 bis 5, **dadurch gekennzeichnet, dass** R² eine lineare oder verzweigte C₆₋₉-Alkandiylgruppe darstellt.

7. Prozess nach einem der Ansprüche 1 bis 6, **dadurch gekennzeichnet, dass** R² eine 1,8-Octandiylgruppe darstellt.

8. Prozess nach einem der Ansprüche 1 bis 7, **dadurch gekennzeichnet, dass** eine Verbindung der Formel (I) aus der Gruppe bestehend aus 12-Oxabicyclo[6.3.1]dodec-8-en, 10-Methyl-12-oxabicyclo[6.3.1]dodec-8-en, 13-Oxabicyclo[7.3.1]tridec-9-en, 11-Methyl-13-oxabicyclo[7.3.1]tridec-9-en, 14-Oxabicyclo[8.3.1]tetradec-10-en, 12-Methyl-14-oxabicyclo[8.3.1]tetradec-10-en, 15-Oxabicyclo[9.3.1]pentadec-11-en, 13-Methyl-15-oxabicyclo[9.3.1]pentadec-11-en, 16-Oxabicyclo[10.3.1]hexadec-12-en, 14-Methyl-16-oxabicyclo[10.3.1]hexadec-12-en, 17-Oxabicyclo[11.3.1]heptadec-13-en und 15-Methyl-17-oxabicyclo[11.3.1]heptadec-13-en ausgewählt sein kann.

9. Prozess nach einem der Ansprüche 1 bis 8, **dadurch gekennzeichnet, dass** eine Verbindung der Formel (II) aus der Gruppe bestehend aus Cycloundecan-1,5-dion, 3-Methylcycloundecan-1,5-dion, Cyclododecan-1,5-dion, 3-Methylcyclododecan-1,5-dion, Cyclotridecan-1,5-dion, 3-Methylcyclotridecan-1,5-dion, Cyclotetradecan-1,5-dion, 3-Methylcyclotetradecan-1,5-dion, Cyclopentadecan-1,5-dion, 3-Methylcyclopentadecan-1,5-dion, Cyclohexadecan-1,5-dion und 3-Methylcyclohexadecan-1,5-dion ausgewählt sein kann.

10. Prozess nach einem der Ansprüche 1 bis 9, **dadurch gekennzeichnet, dass** der Metallkatalysator von der folgenden Formel ist
[M(O)m(X)n] (IV)
wobei M ein Metall ist, ausgewählt aus der Gruppe bestehend aus Übergangsmetall oder Nachübergangsmetall, m 0 oder 1 oder 2 ist, X einen anionischen Liganden darstellt; und n eine ganze Zahl zwischen 1 und 6 ist.

11. Prozess nach Anspruch 10, **dadurch gekennzeichnet, dass** M aus der Gruppe bestehend aus Aluminium, Titan, Zirkonium und Vanadium ausgewählt ist.

12. Prozess nach einem der Ansprüche 1 bis 11, **dadurch gekennzeichnet, dass** der Metallkatalysator aus der Gruppe bestehend aus [Al(OR)₃], [Al(OAc)₃], [Al(acac)₃], [Ti(OR)₄], [Ti(O)(acac)₂], [Zr(OR)₄], [Zr(acac)₄], [V(O)(acac)₂], [V(acac)₃] und [V(O)(OR)₃]; R ausgewählt ist und eine C₁₋₆-Alkylgruppe ist.

13. Prozess nach einem der Ansprüche 1 bis 12, **dadurch gekennzeichnet, dass** die Wasserstoffquelle ein Kohlenwasserstoff ist, der mindestens eine sekundäre alkoholfunktionelle Gruppe umfasst und einen Siedepunkt gleich oder größer als 80°C aufweist.

14. Prozess nach einem der Ansprüche 1 bis 13, **dadurch gekennzeichnet, dass** die Wasserstoffquelle von der folgenden Formel ist 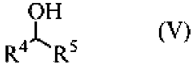 wobei R⁴ und R⁵ unabhängig voneinander eine lineare C₁₋₁₀-Alkylgruppe, die gegebenenfalls durch eine Hydroxygruppe oder eine Arylgruppe substituiert ist, eine lineare C₂₋₁₀-Alkenylgruppe, die gegebenenfalls durch eine Hydroxygruppe oder eine Arylgruppe substituiert ist, eine verzweigte oder cyclische C₃₋₁₀-Alkyl- oder Alkenylgruppe, die gegebenenfalls durch eine Hydroxygruppe oder eine Arylgruppe substituiert ist, oder eine Phenylgruppe, die gegebenenfalls durch eine bis fünf C₁₋₃-Alkyl- oder Alkoxygruppen, Hydroxygruppen oder Halogenatome substituiert ist, darstellen; oder R⁴ und R⁵zusammengenommen ein lineares oder verzweigtes C₂₋₁₀-Alkandiyl oder Alkenindiyl, das gegebenenfalls durch eine Hydroxygruppe oder eine Arylgruppe substituiert ist, darstellen.

15. Prozess nach einem der Ansprüche 1 bis 14, **dadurch gekennzeichnet, dass** die Wasserstoffquelle 1-Phenylethan-1-ol, 2-Methyl-2,4-pentandiol, Cyclohexanol, 4-Methylpentan-2-ol, Cyclopentanol oder Octan-2-ol ist.

16. Prozess nach einem der Ansprüche 1 bis 15, **dadurch gekennzeichnet, dass** der Prozess in Gegenwart eines Co-Katalysators durchgeführt wird, vorzugsweise der Co-Katalysator gegebenenfalls substituierte Phenole oder gegebenenfalls substituierte Bisphenole ist.

17. Prozess nach einem der Ansprüche 1 bis 16, **dadurch gekennzeichnet, dass** die Verbindung (I) 14-Methyl-16-oxabicyclo[10.3.1]hexadec-12-en ist und die Verbindung (II) 3-Methylcyclopentadecan-1,5-dion ist.

18. Prozess nach Anspruch 17, **dadurch gekennzeichnet, dass** der Prozess ferner den Schritt des Umwandelns von 14-Methyl-16-oxabicyclo[10.3.1]hexadec-12-en in 3-Methyl-5-cyclopentadecen-1-on umfasst.

## Revendications

1. Procédé de préparation d'un composé de formule (1) 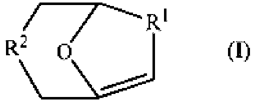
sous la forme de l'un quelconque de ses stéréoisomères ou d'un mélange de ceux-ci et dans lequel R¹ représente un groupe alcanediyle ou alcènediyle en C₁₋₅ linéaire ou ramifié, facultativement substitué par un groupe phényle et R² représente un groupe alcanediyle ou alcènediyle en C₁₋₁₀ linéaire ou ramifié, facultativement substitué par un groupe phényle ;
comprenant la réaction d'un composé de formule (II) 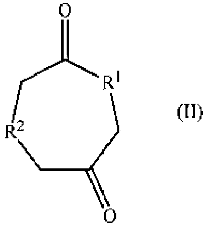
sous la forme de l'un quelconque de ses stéréoisomères et dans lequel R¹ et R² ont la même signification que celle définie dans la formule (1) ;
le procédé étant une réaction du type réduction et cyclisation-déshydratation dans un pot du composé de formule (II) dans des conditions d'hydrogénation par transfert ;
avec un catalyseur métallique à condition qu'un catalyseur au ruthénium soit exclu, et en présence d'une source d'hydrogène ;
dans lequel le catalyseur métallique est présent dans une concentration allant de 1000 ppm à 15 000 ppm par rapport à la quantité totale de substrat.

2. Procédé selon la revendication 1, **caractérisé en ce que** R¹ représente un groupe alcanediyle en C₁₋₄ linéaire ou ramifié.

3. Procédé selon la revendication 1 ou 2, **caractérisé en ce que** R¹ représente un groupe alcanediyle en C₂₋₃ linéaire ou ramifié.

4. Procédé selon l'une quelconque des revendications 1 à 3, **caractérisé en ce que** R¹ représente un groupe 1,2-propanediyle ou un groupe 1,2-éthanediyle.

5. Procédé selon l'une quelconque des revendications 1 à 4, **caractérisé en ce que** R² représente un groupe alcanediyle en C₄₋₉ linéaire ou ramifié.

6. Procédé selon l'une quelconque des revendications 1 à 5, **caractérisé en ce que** R² représente un groupe alcanediyle en C₆₋₉ linéaire ou ramifié.

7. Procédé selon l'une quelconque des revendications 1 à 6, **caractérisé en ce que** R² représente un groupe 1,8-octanediyle.

8. Procédé selon l'une quelconque des revendications 1 à 7, **caractérisé en ce que** le composé de formule (I) peut être choisi dans le groupe constitué de : 12-oxabicyclo[6.3.1]dodéc-8-ène, 10-méthyl-12-oxabicyclo[6.3.1]dodéc-8-ène, 13-oxabicyclo[7.3.1]tridéc-9-ène, 11-méthyl-13-oxabicyclo[7.3.1]tridéc-9-ène, 14-oxabicyclo[8.3.1]tétradéc-10-ène, 12-méthyl-14-oxabicyclo[8.3.1]tétradéc-10-ène, 15-oxabicyclo[9.3.1]pentadéc-11-ène, 13-méthyl-15-oxabicyclo[9.3.1]pentadéc-11-ène, 16-oxabicyclo[10.3.1]hexadéc-12-ène, 14-méthyl-16-oxabicyclo[10.3.1]hexadéc-12-ène, 17-oxabicyclo[11.3.1]heptadéc-13-ène et 15-méthyl-17-oxabicyclo[11.3.1]heptadéc-13-ène.

9. Procédé selon l'une quelconque des revendications 1 à 8, **caractérisé en ce que** le composé de formule (II) peut être choisi dans le groupe constitué de : cycloundécane-1,5-dione, 3-méthylcycloundécane-1,5-dione, cyclododécane-1,5-dione, 3-méthylcyclododécane-1,5-dione, cyclotridécane-1,5-dione, 3-méthylcyclotridécane-1,5-dione, cyclotétradécane-1,5-dione, 3-méthylcyclotétradécane-1,5-dione, cyclopentadécane-1,5-dione, 3-méthylcyclopentadécane-1,5-dione, cyclohexadécane-1,5-dione et 3-méthylcyclohexadécane-1,5-dione.

10. Procédé selon l'une quelconque des revendications 1 à 9, **caractérisé en ce que** le catalyseur métallique est de formule
[M (O) m (X) n] (IV)
dans laquelle M est un métal choisi dans le groupe constitué d'un métal de transition ou d'un métal post-transition, m est 0, ou 1 ou 2, X représente un ligand anionique ; et lorsque n est un entier compris entre 1 et 6.

11. Procédé selon la revendication 10, **caractérisé en ce que** M est choisi dans le groupe constitué de l'aluminium, du titane, du zirconium et du vanadium.

12. Procédé selon l'une quelconque des revendications 1 à 11, **caractérisé en ce que** le catalyseur métallique est choisi dans le groupe constitué de [Al(OR)₃], [Al(OAc)₃], [Al(acac)₃], [Ti(OR)₄], [Ti(O)(acac)₂], [Zr(OR)₄], [Zr(acac)₄], [V(O)(acac)₂], [V(acac)₃] et [V(O)(OR)₃]; R étant un groupe alkyle en C₁₋₆.

13. Procédé selon l'une quelconque des revendications 1 à 12, **caractérisé en ce que** la source d'hydrogène est un hydrocarbure comprenant au moins un groupe fonctionnel alcool secondaire et ayant un point d'ébullition égal ou supérieur à 80°C.

14. Procédé selon l'une quelconque des revendications 1 à 13, **caractérisé en ce que** la source d'hydrogène est de formule 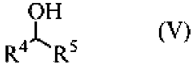 dans laquelle R⁴ et R⁵, indépendamment l'un de l'autre, représentent un groupe alkyle linéaire en C₁₋₁₀ facultativement substitué par un groupe hydroxy ou un groupe aryle, un groupe alcényle linéaire en C₂₋₁₀ facultativement substitué par un groupe hydroxy ou un groupe aryle, un groupe alkyle ou alcényle ramifié ou cyclique en C₃₋₁₀ facultativement substitué par un groupe hydroxy ou un groupe aryle, ou un groupe phényle facultativement substitué par un à cinq groupes alkyle ou alcoxy en C₁₋₃, groupes hydroxy ou atomes d'halogène ; ou R⁴ et R⁵, conjointement, représentent un alcanediyle ou alcénediyle en C₂₋₁₀ linéaire ou ramifié facultativement substitué par un groupe hydroxy ou un groupe aryle.

15. Procédé selon l'une quelconque des revendications 1 à 14, **caractérisé en ce que** la source d'hydrogène est le 1-phényléthan-1-ol, le 2-méthyl-2,4-pentanediol, le cyclohexanol, le 4-méthylpentan-2-ol, le cyclopentanol ou l'octan-2-ol.

16. Procédé selon l'une quelconque des revendications 1 à 15, **caractérisé en ce que** le procédé est réalisé en présence d'un co-catalyseur, de préférence le co-catalyseur est des phénols facultativement substitués ou des bisphénols facultativement substitués.

17. Procédé selon l'une quelconque des revendications 1 à 16, **caractérisé en ce que** le composé (1) est le 14-méthyl-16-oxabicyclo[10.3.1]hexadéc-12-ène et le composé (II) est la 3-méthylcyclopentadécane-1,5-dione.

18. Procédé selon la revendication 17, **caractérisé en ce qu'**il comprend en outre l'étape de conversion de 14-méthyl-16-oxabicyclo[10.3.1]hexadéc-12-ène en 3-méthyl-5-cyclopentadécén-1-one.
